# EUROPÄISCHE PATENTSCHRIFT

(11) **EP 0 820 754 B1**
(45) Veröffentlichungstag und Bekanntmachung des Hinweises auf die Patenterteilung: **21.02.2007**
(21) Anmeldenummer: 97110883.2
(22) Anmeldetag: 02.07.1997
(51) Int. Cl.: A61K 6/02

(54) **Verfahren zur Herstellung eines vollkeramischen Dentalaufbaus**
Method for manufacturing an all-ceramic dental structure
Procédé de fabrication d'une structure dentaire entièrement céramique

(30) Priorität: 26.07.1996 DE 19630412
(43) Veröffentlichungstag der Anmeldung: 28.01.1998
(73) Patentinhaber: Ivoclar Vivadent AG, 9494 Schaan (LI)
(72) Erfinder: Foser, Hans Peter, FL-9496 Balzers (LI)
(74) Vertreter: Baronetzky, Klaus

(56) Entgegenhaltungen:
- EP-A- 0 631 995
- DE-A- 4 019 846
- DE-A- 4 404 921
- DE-C- 4 423 794
- DATABASE WPI Section Ch, Week 198910 Derwent Publications Ltd., London, GB; Class D21, AN 1989-073148 XP002146424 & JP 01 025849 A (OLYMPUS OPTICAL CO LTD), 27. Januar 1989 (1989-01-27)

## Beschreibung

Die Erfindung betrifft ein Verfahren zur Herstellung eines vollkeramischen Dentalaufbaus aus einem ZrO₂-Stift und einer ZrO₂-Glaskeramik, einen vollkeramischen Dentalaufbau aus diesen Materialien, sowie dessen Verwendung.

Bislang wurden für dentale Stiftkonstruktionen bzw. Dentalaufbauten aus werkstoffkundlichen und herstellungsbedingten Gründen metallische Stifte und Aufbaumaterialien verwendet. Derartige herkömmliche Stiftsysteme sind beispielsweise aus: M. Simon, "Neue Perspektiven zur vollkeramischen Stabilisierung und zum Aufbau devitaler Zähne", Quintessenz 46, Seiten 1085 - 1101, (1995) und aus: D. Kaelin und P. Schärer, "Aufbausysteme in der Kronen- und Brückenprothetik", Schweiz. Monatsschr. Zahnmed. Vol. 101. S. 457-463 (1991) bekannt geworden. Die Verblendung der Aufbauten erfolgt separat mit Kunststoff oder Keramik. Die bekannten Lösungen weisen jedoch eine Reihe von Problemen auf, bspw. die Korrosion bestimmter Metalle bzw. Metallegierungen und deren Ablagerung im umliegenden Gewebe, Entzündungen der Gingiva oder dunkle Verfärbungen der umgebenden Hart- und Weichgewebe aufgrund der opaken Beschaffenheit metallischer Materialien. Deshalb wurde in den letzten Jahren zunehmend versucht, von Metallaufbauten Abstand zu nehmen und zu durchscheinenden Materialien mit besserer Optik und Biokompatibilität überzugehen.

Eine besonders hohe mechanische Festigkeit weisen dabei die Zirkondioxid-Stifte auf, wie sie beispielsweise in K. H. Meyenberg, H. Lüthy und P. Schärer, "Zirconia Posts: A New All-Ceramic Concept for Nonvital Abutment Teeth", Journal of Esthetic Dentistry, Vol. 7, Nr. 2, (1995) und der vorstehend genannten Literaturstelle Quintessenz 46, 1085 - 1101, (1995) beschrieben sind. Zur Herstellung von Dentalaufbauten wird der ZrO₂-Wurzelstift eingesetzt und ein Aufbau aus einem Composit vorgenommen. Im Labor wird dann eine Krone, beispielsweise aus IPS-Empress-Keramik^{®} (Fa. Ivoclar, Schaan, Liechtenstein) hergestellt und in herkömmlicher Weise, z.B. mit einem Adhesiv-System und einem durchscheinenden Kompositzement aufzementiert. Derartige Lösungen weisen zwar eine gute Biokompatibilität auf, nachdem metallische Werkstoffe vermieden werden können. Jedoch wäre eine verbesserte Festigkeit wünschenswert.

Aus der US-4,936,776 sind verschiedene Dentalaufbauten aus durchscheinenden Porzellankeramikmaterialien bekannt. Nach einer Ausführungsform wird auf einen durchscheinenden Keramikstift eine Porzellankeramik aufgebrannt. Weiterhin hat auch der in der US-4,936,775 offenbarte Aufbau mit aufgebrannter Porzellankeramikkrone aufgrund mangelnder Bruchfestigkeit eine geringe Belastbarkeit.

Aus der DE 44 23 794 C1 ist eine ZrO₂-haltige Glaskeramik bekannt, die neben einer sehr hohen Festigkeit eine gute Verarbeitbarkeit bei relativ niedrigen Temperaturen aufweist. Darüberhinaus bietet sie einen sehr guten Haftgrund für gesinterte ZrO₂-Keramiken, was sie für Dentalprodukte gut einsetzbar macht.

Es ist auch bereits vorgeschlagen worden, auf die bekannten Metallstifte einen keramischen Aufbau aufzubrennen und eine vollkeramische Krone darauf aufzuzementieren. Dabei besteht jedoch das Problem von Spannungsrissen in der Keramik, wie es auch in der vorgenannten Literaturstelle Schweiz. Monatsschr. Zahnmed. Vol. 101, (1991) beschrieben ist.

Aus der DE 40 19 846 A1 ist eine künstliche Zahnwurzel bekannt, bei der in der Mitte eines im Wesentlichen aus Hydroxylapatit bestehenden Hauptteils ein Aufbaupfosten aus gesintertem Aluminium- oder Zirkonoxid eingebettet ist. Durch die unterschiedlichen Materialien bestehen auch bei dieser Lösung die schon genannten Probleme unterschiedlicher Wärmedehnungskoeffizienten.

Desweiteren wird in der Veröffentlichung "Database WPI Section CH", Week 198910, Derwent Publications Ltd., London, GB; Class D21, AN 1989-073148 & JP 01 025849 A (Olympus Co Ltd) (1989-01-27) eine sehr haltbare Dentalwerkstoffkombination aus gesintertem Aluminium oder Zirkonium und einer Keramik beschrieben, wobei der Stift aus dem Sinterwerkstoff den Keramikkörper trägt. Auch bei dieser Lösung können aufgrund der unterschiedlichen Werkstoffe die oben genannten Problem auftreten.

Demgegenüber ist es Aufgabe der Erfindung, unter Vermeidung der Nachteile des Standes der Technik ein Verfahren zur Herstellung eines vollkeramischen Dentalaufbaus aus einem hochfesten Stift, insbesondere einem Zr02-Stift, und einer auf diesen aufgepressten Keramik, insbesondere einer ZrO2-Glaskeramik, mit besonders guten mechanischen Eigenschaften bereitzustellen, wobei besonders stabile und einen ausgezeichneten Haftverbund bildende Dentalaufbauten mit ausgezeichneten Festigkeiten, insbesondere Gesamt-Biegefestigkeiten und Zugfestigkeiten hergestellt werden können, die keine Spannungsrisse aufweisen.

Diese Aufgabe wird erfindungsgemäß durch das Verfahren gemäss Anspruch 1 gelöst. Vorteilhafte Weiterbildungen ergeben sich aus den Unteransprüchen.

Ein besonderer Vorteil der Erfindung liegt darin, daß eine sehr gute Gesamtstabilität des Dentalaufbaus erzielt wird, ohne daß auf die bisher verwendeten hochfesten Metallstifte mit ihren vorgenannten Nachteilen, wie ungenügende biologische Verträglichkeit, schlechte Ästhetik, Korrosion, etc., zurückgegriffen werden muß.

Die erfindungsgemäß hergestellten Dentalaufbauten weisen neben biologischer Unbedenklichkeit bzw. hoher Biokompatibilität eine ausgezeichnete, dem natürlichen Zahn angeglichene Ästhetik und eine sehr große Haltbarkeit auf. Die hervorragenden physikalischen Eigenschaften sind auf mehrere Faktoren zurückzuführen. Zunächst wird durch die gezielte Verwendung von ZrO₂-Stiften eine besonders biegefeste Stabilisierung der Dentalaufbauten in der Zahnwurzel ermöglicht. Weiterhin weist durch die Verwendung von ZrO₂-Glaskeramiken auch der der Zahnkrone zugewandten Teil des Dentalaufbaus eine besonders hohe mechanische Belastbarkeit auf.

Schließlich ermöglicht die erfindungsgemäße Abstimmung der Wärmeausdehnungskoeffizienten des ZrO₂-Stifts und der ZrO₂-Glaskeramik aufgrund der Materialkompatibilität zueinander in Verbindung mit dem Aufpressen eine besonders innige und stabile Verbindung.

Dadurch wird im Vergleich zum herkömmlichen adhäsiven Verbinden bzw. Aufzementieren besonders die Gesamtstabilität des Dentalaufbaus erheblich erhöht. Es wird nicht nur die gute Haftfähigkeit zwischen ZrO₂ und ZrO₂-haltigen Glaskeramiken ausgenutzt. Da das Aufbringen der ZrO₂-Glaskeramik unter Druckanwendung erfolgt, wird die innige Verbindung der beiden Komponenten verstärkt. Dieses Anpressen der ZrO₂-Glaskeramik kann erfindungsgemäß nur dann seine Wirkung entfalten, wenn der Wärmeausdehnungskoeffizient des ZrO₂-Stifts mindestens demjenigen der ZrO₂-Glaskeramik entspricht.

Ein besonders vorteilhafter und überraschender Effekt ergibt sich dadurch, daß sich der ZrO₂-Stift beim Abkühlen aufgrund des höheren Wärmeausdehnungskoeffizienten stärker zusammenzieht als die umgebende ZrO₂-Glaskeramik. Dadurch entsteht, nachdem die ZrO₂-Glaskeramik auf den ZrO₂-Stift aufgepreßt wurde, eine gewisse Spannung zwischen den beiden Komponenten, wobei die auf den ZrO₂-Stift aufgepreßte ZrO₂-Glaskeramik durch die Kontraktion des Stiftes nach der Art von Spannbeton unter Druck gesetzt wird.

Es hat sich überraschenderweise herausgestellt, daß dadurch die mechanischen Eigenschaften des vollkeramischen Metallaufbaus verbessert werden. So weisen die erfindungsgemäßen Dentalaufbauten eine hervorragende Gesamtbiegefestigkeit, eine Spannungsrißfreiheit, sowie allgemein eine besonders hohe Widerstandskraft gegen die in der Praxis auf den eingesetzten Dentalaufbau einwirkenden Kräfte auf. Überraschenderweise ist die erfindungsgemäße Festigkeit trotz unterschiedlicher Materialwahl für den ZrO₂-Stift und den ZrO₂-Glaskeramikaufbau - wie nachstehend ausgeführt - gewährleistet. Unter Gesamtbiegefestigkeit versteht man die Festigkeit, die ein ZrO₂-Stift mit einem darauf aufgebrannten Aufbau aus einer ZrO₂-Glaskeramik besitzt.

Ab einem gewissen Abstand zwischen den Wärmeausdehnungskoeffizienten von ZrO₂-Stift und ZrO₂-Glaskeramik können beim Aufpressen Risse entstehen. Dieser "kritische" Abstand der Wärmeausdehnungskoeffizienten ist im Einzelfall von der bestimmten verwendeten ZrO₂-Glaskeramik und dem bestimmten verwendeten ZrO₂-Stift abhängig. Allgemein treten diese Probleme jedoch auf, wenn der Wärmeausdehnungskoeffizient des ZrO₂-Stifts mehr als etwa 2 µm/m·K über dem Wärmeausdehnungskoeffizienten der ZrO₂-Glaskeramik liegt oder der Unterschied zwischen den Wärmeausdehnungskoeffizienten allgemein größer als 2 µm/m·K ist.

Ein weiterer Vorteil der Erfindung liegt in der Zeitersparnis, die sich durch die Herstellung des gesamten Dentalaufbaus während eines Brennvorgangs ergibt. Dadurch wird ein getrenntes Aufzementieren bzw. Aufkleben des Keramikaufbaus auf den Wurzelstift vermieden. Außerdem können somit eventuelle Unverträglichkeitsreaktionen gegenüber den dazu verwendeten Materialien vermieden werden. Schließlich weisen die erfindungsgemäß hergestellten Dentalaufbauten eine besonders gute Asthetik auf, da sie, ähnlich einem natürlicher Zahn, eine durchgehend transluzente Struktur in einem Stück darstellen und der Optik des natürlichen Dentins bzw. des Schmelzes gut angepaßt sind.

Unter Dentalaufbau wird hierbei jede beliebig geformte Konstruktion verstanden, die eine Kombination eines präfabrizierten oder individuell abgeformten Stifts mit einem beliebig in Abhängigkeit von der Verwendung geformten Aufbaus, bspw. auch eine Teil- oder Vollkrone darstellt.

Es hat sich herausgestellt, daß sich die erfindungsgemäßen Vorteile besonders gut verwirklichen lassen, wenn der Wärmeausdehnungskoeffizient des ZrO₂-Stifts 0,5 bis 2,0 µm/m·K über dem Wärmeausdehnungskoeffizienten der ZrO₂-Glaskeramik liegt.

Nach einer bevorzugten Ausführungsform weist der ZrO₂-Stift einen Wärmeausdehnungskoeffizienten von etwa 11 µm/m·K, und die ZrO₂-Glaskeramik einen Wärmeausdehnungskoeffizienten von etwa 9,5 µm/m·K auf.

Erfindungsgemäß werden unter ZrO₂-Stiften vollkeramische (Wurzel-)Stifte aus Zirkondioxid verstanden, die bspw. auch mit Yttriumoxid (etwa 3-4 Gew.-%. Y₂O₃) teilstabilisiert sein können. Derartige Keramiken ergeben eine besonders gute Haftung zu den ZrO₂-haltigen Glaskeramiken.

Bei der vorliegenden Erfindung können weiterhin beliebige ZrO₂-haltige Glaskeramiken mit ausreichenden mechanischen Eigenschaften und abgestimmten Wärmeausdehnungskoeffizient-Werten verwendet werden. Beispielhaft seien die in der DE-C1-44 23 794, auf die vollinhaltlich Bezug genommen wird, offenbarten ZrO₂-Glaskeramiken genannt.

Bei einer bevorzugten Ausführungsform erfolgt das Aufpressen der ZrO₂-Glaskeramik in einem Keramikpreßverfahren, wobei das ZrO₂-Glaskeramikmaterial zunächst in Form eines Rohlings vorliegen kann.

Es stellte sich heraus, daß bei der vorliegenden Erfindung besonders gute Ergebnisse erzielt werden, wenn beim Keramikpreßverfahren das ZrO₂-Glaskeramikmaterial als Rohling vorliegt und durch Erhitzen plastifiziert, unter Druckanwendung in die Hohlform gepreßt, gesintert und beim Abkühlen ausgehärtet wird. Dadurch ist sichergestellt, daß das ZrO₂-Glaskeramikmaterial im plastifizierten Zustand nicht nur besonders gut an den ZrO₂-Stift angepreßt wird, sondern sich auch während des Sinterns und Abkühlens die innige Verbindung ausbildet und sich die vorbeschriebenen vorteilhaften Effekte unter Druckanwendung einstellen können.

Das in der EP-A1-0 231 773 beschriebene Keramikpreßverfahren und der dabei verwendete spezielle Ofen liefert bei dem erfindungsgemäßen Verfahren sehr gute Ergebnisse. Bezüglich einer genauen Beschreibung des Verfahrens bzw. des verwendeten Ofens wird ausdrücklich auf die EP-A1-0 231 773 verwiesen. Ein derartiges Verfahren bzw. eine derartige Vorrichtung finden bspw. auch im IPS-Empress^{®}-Verfahren der Fa. Ivoclar, Schaan, Liechtenstein, Anwendung.

Bspw. kann ein typisches erfindungsgemäßes Verfahren die folgenden Verfahrensschritte aufweisen, wobei je nach Verwendungszweck selbstverständlich Modifikationen vorgenommen werden können:
1. Abdrucknahme der Mundsituation mit eingesetztem keramischem Wurzelstift;
2. Herstellung eines Modells aus dem Abdruck, wobei der Stift aus dem Modell herausragt;
3. Modellation einer Rekonstruktion aus einem ausbrennbaren Material, vorzugsweise Wachs oder Kunststoff, am Stift (bspw. in Form eines Kronenstumpfs oder einer Teil- oder Vollkrone);
4. Ansetzen eines Wachsstiftes bzw. -strangs, der später als Guß- bzw. Preßkanal dient;
5. Einbetten von Stift und angewachsten Teil in eine Muffel mit einer aushärtbaren Einbettmasse;
6. Entfernen des Wachses bzw. des ausbrennbaren Materials durch Erhitzen;
7. Durchführen des eigentlichen Keramikpreßverfahrens, bspw. gemäß der EP-0 231 773, wobei das ZrO₂-Glaskeramikmaterial über den Gußkanal (zunächst als Rohling) eingebracht, durch Erhitzen plastifiziert und über einen im Gußkanal angeordneten Kolben unter Druck angepreßt und während des Abkühlens ausgehärtet wird, wobei der ZrO₂-Stift höchstens einen um den Wert 2 höheren Wärmeausdehnungskoeffizienten als die ZrO₂-Glaskeramik aufweist.

Die Druckausübung kann mit konstanter oder veränderliche Stärke oder auch stoßweise erfolgen.

Besonders günstig ist es, daß bei dem erfindungsgemäßen Verfahren relativ niedrige Brenntemperaturen verwendet werden können, die im allgemeinen unter 1200°C, bevorzugt sogar unter 1000°C liegen. Dadurch wird eine energetisch günstige und damit auch kostengünstige Verarbeitung ermöglicht. Weiterhin sind weniger aufwendige Brennöfen ausreichend, und die Aufheizzeit ist reduziert.

Bei einer bevorzugten Ausführungsform der Erfindung weist der Zr02-Stift eine Biegefestigkeit von mindestens 600 MPa, insbesondere von 800 bis 1500 MPa auf. Damit liegen die verwendeten ZrO₂-Stifte in ihrer Biegefestigkeit im Bereich hochfester, herkömmlich verwendeter Metallstifte, oder sogar darüber. Bei den erfindungsgemäss hergestellten vollkeramischen Dentalaufbauten kommt diese hohe Biegefestigkeit insbesondere der Gesamtbiegefestigkeit des Dentalaufbaus zugute, so dass vorzugsweise Gesamtbiegefestigkeiten von mindestens 100 MPa erreicht werden.

Nach einem weiteren Aspekt betrifft die Erfindung einen vollkeramischen Dentalaufbau nach Anspruch 13, der vorzugsweise nach einem Verfahren gemäss einem der Ansprüche 1 bis 12 hergestellt werden kann.

Nach einem weiteren Aspekt betrifft die vorliegende Erfindung die Verwendung eines vollkeramischen Dentalaufbaus gemäss Anspruch 14 als Dentalprodukt. Darunter fällt allgemein jede mögliche und in der Zahnheilkunde sinnvolle Verwendung, bspw. der Wiederaufbau fehlender Zahnhartsubstanz bei vitalen oder devitalen Zähnen. Übliche Einsatzgebiete solcher Dentalaufbauten sind bspw. Zahnwurzelkonstruktionen mit Teil- bzw. Vollkronen.

Im folgenden wird die Erfindung anhand eines Ausführungsbeispiels näher erläutert.

### Beispiel

Es wurde ein Biopost TM -Wurzelstift (Fa. Incermed S.A., Lausanne) der passenden Grösse aus Zirkondioxid verwendet. Der Stift wurde in den auf herkömmliche Weise vorbereiteten Wurzelkanal eingesetzt und ein Abdruck der Mundsituation genommen. Anschließend wurde aus dem Abdruck ein Modell aus Superhartgips hergestellt, wobei der Stift aus dem Modell herausragte. Auf der Grundlage dieses Modells wurde eine Krone aus einem rückstandslos verbrennenden Wachs am Stift modelliert, wobei der Stift mit Al₂O₃ konditioniert wurde.

Das modellierte Objekt wurden auf einer IPS-Empress^{®}-Muffelbasis (Fa. Ivoclar) angesetzt. Es können auch mehrere Objekte auf eine Muffelbasis gesetzt werden, wobei der Abstand zwischen den Objekten bei dieser Technik mindestens 3 mm betragen sollte. Anschließend wurde die Muffelbasis mit einem Papierzylinder umhüllt und die Modellation einschließlich ZrO₂-Stift in eine IPS-Empress-Spezialeinbettmasse eingebettet. Nach der vorgeschriebenen Abbdindezeit wurde die Muffellehre und die Muffelbasis mit einer Drehbewegung entfernt und die Papiermanschette abgenommen. Dabei wurde darauf geachtet, daß keine Einbettmassenreste in den Preßkanal gelangen.

Die vorbereitete Muffel wurden in einen Vorwärmofen gestellt und zusammen mit einem AlOx-Kolben, wie in der IPS-Empress-Verarbeitungsanleitung der Fa. Ivoclar angegeben, vorgewärmt. Der ZrO₂-Rohling wurde separat vorgewärmt. Bei der ZrO₂-Glaskeramik handelte es sich um die in der DE-44 23 794 als Beispiel 1 angegebene Zusammensetzung mit folgenden Gewichtsprozentanteilen (in Klammern): SiO₂ (52,8), Al₂O₃ (3,0), Li₂O (12,9), P₂O₅ (10,4), ZrO₂ (20,9). Nach Ablauf der Vorwärmphase wurde der ZrO₂-Glaskeramikrohling und anschließend der AlOx-Kolben in den Gußkanal eingesetzt und das Keramikpreßverfahren bei 950°C und 5 bar durchgeführt. Nach dem Abkühlen wurde das Objekt auf herkömmliche Weise von der Muffel getrennt und gereinigt und konnte, ggf. nach kleinen Formkorrekturen, Bemalung und Glasieren, direkt in die Zahnkavität eingesetzt werden.

## Patentansprüche

1. Verfahren zur Herstellung eines vollkeramischen Dentalaufbaus, der aus einem ZrO₂-Stift und einer ZrO₂-Glaskeramik hergestellt wird, wobei die ZrO₂-Glaskeramik auf den ZrO₂-Stift aufgepresst wird, **dadurch gekennzeichnet, dass** der Wärmeausdehnungskoeffizient des ZrO₂-Stifts dem Wärmeausdehnungskoeffizienten der ZrO₂-Glaskeramik, gemessen bei 20 - 500 °C, entspricht oder bis 3,0 µm/m.K darüber liegt.

2. Verfahren nach Anspruch 1, **dadurch gekennzeichnet, dass** der Stift ein Keramikstift ist.

3. Verfahren nach Anspruch 1, **dadurch gekennzeichnet, dass** der Wärmeausdehnungskoeffizient des ZrO₂-Stifts, gemessen bei 20 - 500 °C, 0,5 bis 2,0 µm/m·K über dem Wärmeausdehnungskoeffizienten der Zr02-Glaskeramik liegt.

4. Verfahren gemäss einem der vorhergehenden Ansprüche, **dadurch gekennzeichnet, dass** der ZrO₂-Stift einen Wärmeausdehnungskoeffizient von etwa 11 µm/m·K, und die ZrO₂-Glaskeramik einen Wärmeausdehnungskoeffizienten von etwa 9,5 µm/m·K aufweist.

5. Verfahren gemäss einem der vorhergehenden Ansprüche, **dadurch gekennzeichnet, dass** die ZrO₂-Glaskeramik in Form eines Rohlings vorliegt und einen Schmelzpunkt aufweist, der niedriger als der Schmelzpunkt des Stifts ist.

6. Verfahren gemäss einem der vorhergehenden Ansprüche, **dadurch gekennzeichnet, dass** die Glaskeramik in einem Keramikpressverfahren, unmittelbar auf den ZrO₂-Stift aufgebrannt wird.

7. Verfahren gemäss einem der vorhergehenden Ansprüche, **dadurch gekennzeichnet, dass** bei dem Keramikpressverfahren das ZrO₂-Glaskeramikmaterial durch Erhitzen plastifiziert und unter Druckanwendung in eine Hohlform gepresst, gesintert und abgekühlt wird.

8. Verfahren gemäss einem der vorhergehenden Ansprüche, **dadurch gekennzeichnet, dass** bei dem Keramikpressverfahren ein massgenaues Modell des späteren Aufbaus aus dem ZrO₂-Stift und einem ausbrennbaren Material angefertigt und mit einem den späteren Gusskanal bildenden Strang versehen in eine aushärtbare Einbettmasse eingebettet wird, worauf das ausbrennbare Material durch Erhitzen entfernt, das ZrO₂-Glaskeramikmaterial als Rohling über den Gusskanal eingebracht und über einen im Gusskanal angeordneten Kolben mit Druck beaufschlagt und unter thermischer Plastifizierung des Rohlings in eine Hohlform gepresst wird.

9. Verfahren gemäss einem der vorhergehenden Ansprüche, **dadurch gekennzeichnet, dass** die Temperatur beim Pressen 1000 °C nicht übersteigt.

10. Verfahren gemäss einem der vorhergehenden Ansprüche, **dadurch gekennzeichnet, dass** der ZrO₂-Stift ein präfabrizierter, teilstabilisierter ZrO₂-Stift ist.

11. Verfahren gemäss einem der vorhergehenden Ansprüche, **dadurch gekennzeichnet, dass** der ZrO₂-Stift eine Biegefestigkeit von mindestens 600 MPa, insbesondere von 800-1500 MPa, besitzt.

12. Verfahren gemäss einem der vorhergehenden Ansprüche, **dadurch gekennzeichnet, dass** der vollkeramische Dentalaufbau eine Biegefestigkeit von mindestens 100 MPa aufweist.

13. Vollkeramischer Dentalaufbau aus einer ZrO₂-Glaskeramik und einem ZrO₂-Stift, **dadurch gekennzeichnet, dass** der Wärmeausdehnungskoeffizient des ZrO₂-Stifts dem Wärmeausdehnungskoeffizienten der ZrO₂-Glaskeramik entspricht oder bis 2,0 µm/m.K darüber liegt.

14. Verwendung eines vollkeramischen Dentalaufbaus gemäss Anspruch 13 als Dentalprodukt, insbesondere zum Wiederaufbau der fehlenden Zahnhartsubstanz vitaler oder devitaler Zähne, insbesondere als Zahn- und/oder Wurzelrekonstruktion.

## Claims

1. A process for the production of an all-ceramic dental abutment which is produced from a ZrO₂ post and a ZrO₂ vitreous ceramic, the ZrO₂ vitreous ceramic being press-moulded onto the ZrO₂ post, **characterised in that** the coefficient of thermal expansion of the ZrO₂ post matches or is up to 3.0 µm/m·K greater than the coefficient of thermal expansion of the ZrO₂ vitreous ceramic, measured at 20-500°C.

2. A process according to claim 1, **characterised in that** the post is a ceramic post.

3. A process according to claim 1, **characterised in that** the coefficient of thermal expansion of the ZrO₂ post, measured at 20-500°C, is 0.5 to 2.0 µm/m·K greater than the coefficient of thermal expansion of the ZrO₂ vitreous ceramic.

4. A process according to any one of the preceding claims, **characterised in that** the ZrO₂ post exhibits a coefficient of thermal expansion of approx. 11 µm/m·K, and the ZrO₂ vitreous ceramic a coefficient of thermal expansion of approx. 9.5 µm/m·K.

5. A process according to any one of the preceding claims, **characterised in that** the ZrO₂ vitreous ceramic assumes the form of a blank and exhibits a melting point which is lower than the melting point of the post.

6. A process according to any one of the preceding claims, **characterised in that** the vitreous ceramic is fired directly onto the ZrO₂ post in a ceramic pressing process.

7. A process according to any one of the preceding claims, **characterised in that**, in the ceramic pressing process, the ZrO₂ vitreous ceramic material is plasticised by heating and is pressed into a mould cavity with application of pressure, sintered and cooled.

8. A process according to any one of the preceding claims, **characterised in that**, in the ceramic pressing process, a dimensionally accurate model of the subsequent abutment is prepared from the ZrO₂ post and a fusible material and, provided with a strand forming the subsequent casting channel, is embedded in a hardenable embedding composition, whereupon the fusible material is removed by heating, the ZrO₂ vitreous ceramic material is introduced as a blank via the casting channel and is exposed to pressure by a piston arranged in the casting channel and, with thermal plasticisation of the blank, pressed into a mould cavity.

9. A process according to any one of the preceding claims, **characterised in that** the temperature during pressing does not exceed 1000°C.

10. A process according to any one of the preceding claims, **characterised in that** the ZrO₂ post is a prefabricated, partially stabilised ZrO₂ post.

11. A process according to any one of the preceding claims, **characterised in that** the ZrO₂ post has a flexural strength of at least 600 MPa, in particular of 800-1500 MPa.

12. A process according to any one of the preceding claims, **characterised in that** the all-ceramic dental abutment exhibits a flexural strength of at least 100 MPa.

13. An all-ceramic dental abutment made from a ZrO₂ vitreous ceramic and a ZrO₂ post, **characterised in that** the coefficient of thermal expansion of the ZrO₂ post matches or is up to 2.0 µm/m·K greater than the coefficient of thermal expansion of the ZrO₂ vitreous ceramic.

14. Use of an all-ceramic dental abutment according to claim 13 as a dental product, in particular for the restoration of absent dentine in vital or nonvital teeth, in particular as a tooth and/or root reconstruction.

## Revendications

1. Procédé pour la fabrication d'une structure dentaire tout céramique qui est constituée d'un tenon en ZrO₂ et d'une vitrocéramique contenant du ZrO₂, la vitrocéramique contenant du ZrO₂ étant pressée sur le tenon en ZrO₂, **caractérisé en ce que** le coefficient de dilatation thermique du tenon en ZrO₂ correspond au coefficient de dilatation thermique de la vitrocéramique contenant du ZrO₂ mesuré à 20-500 °C ou se situe jusqu'à 3,0 µm/m.K au-dessus de celui-ci.

2. Procédé selon la revendication 1, **caractérisé en ce que** le tenon est un tenon en céramique.

3. Procédé selon la revendication 1, **caractérisé en ce que** le coefficient de dilatation thermique du tenon en ZrO₂, mesuré à 20-500 °C, se situe de 0,5 à 2,0 µm/m.K au-dessus du coefficient de dilatation thermique de la vitrocéramique contenant du ZrO₂.

4. Procédé selon l'une des revendications précédentes, **caractérisé en ce que** le tenon en ZrO₂ présente un coefficient de dilatation thermique d'environ 11 µm/m.K et **en ce que** la vitrocéramique contenant du ZrO₂ présente un coefficient de dilatation thermique d'environ 9,5 µm/m.K.

5. Procédé selon l'une des revendications précédentes, **caractérisé en ce que** la vitrocéramique contenant du ZrO₂ se présente sous la forme d'un lingotin et présente un point de fusion qui est plus faible que le point de fusion du tenon.

6. Procédé selon l'une des revendications précédentes, **caractérisé en ce que** la vitrocéramique est directement cuite sur le tenon en ZrO₂ lors d'un procédé de pressée de la céramique.

7. Procédé selon l'une des revendications précédentes, **caractérisé en ce que**, dans le procédé de pressée de la céramique, la vitrocéramique contenant du ZrO₂ est plastifiée par chauffage et pressée dans une cavité en appliquant une pression, frittée et refroidie.

8. Procédé selon l'une des revendications précédentes, **caractérisé en ce que**, dans le procédé de pressée de la céramique, on fabrique un modèle aux dimensions exactes de la future structure avec le tenon en ZrO₂ et un matériau pouvant brûler, modèle qui est doté d'un fil constituant le futur canal de coulée et qui est noyé dans une masse d'enrobage durcissable, le matériau pouvant brûler étant éliminé par chauffage, la vitrocéramique contenant du ZrO₂ étant insérée sous forme de lingotin par le canal de coulée et introduite sous pression au moyen d'un piston agencé dans le canal de coulée et pressée dans une cavité lors de la plastification thermique du lingotin.

9. Procédé selon l'une des revendications précédentes, **caractérisé en ce que** la température lors du procédé de pressée ne dépasse pas 1000 °C.

10. Procédé selon l'une des revendications précédentes, **caractérisé en ce que** le tenon en ZrO₂ est un tenon en ZrO₂ préfabriqué et partiellement stabilisé.

11. Procédé selon l'une des revendications précédentes, **caractérisé en ce que** le tenon en ZrO₂ présente une résistance à la flexion d'au moins 600 MPa, en particulier de 800 à 1500 MPa.

12. Procédé selon l'une des revendications précédentes, **caractérisé en ce que** la structure dentaire tout céramique présente une résistance à la flexion d'au moins 100 MPa.

13. Structure dentaire tout céramique fabriquée à partir d'une vitrocéramique contenant du ZrO₂ et d'un tenon en ZrO₂, **caractérisée en ce que** le coefficient de dilatation thermique du tenon en ZrO₂ correspond au coefficient de dilatation thermique de la vitrocéramique contenant du ZrO₂ ou se situe jusqu'à 2,0 µm/m.K au-dessus de celui-ci.

14. Utilisation d'une structure dentaire tout céramique selon la revendication 13 comme produit dentaire, en particulier pour la restauration des tissus dentaires durs absents, des dents vitales ou dévitalisées, en particulier comme reconstruction dentaire et/ou radiculaire.
